(19) Europäisches Patentamt
European Patent Office
Office européen des brevets

(11) Publication number: 0 372 870 A2

# (12) EUROPEAN PATENT APPLICATION

(21) Application number: 89312588.0

(22) Date of filing: 01.12.89

(51) Int. Cl.5: C07D 339/06, A01N 43/28

(30) Priority: 05.12.88 GB 8828326

(43) Date of publication of application: 13.06.90 Bulletin 90/24

(84) Designated Contracting States: AT BE CH DE ES FR GB GR IT LI LU NL SE

(71) Applicant: THE WELLCOME FOUNDATION LIMITED
183-193 Euston Road
London NW1 2BP(GB)

Applicant: THE REGENTS OF THE UNIVERSITY OF CALIFORNIA
300 Lakeside Drive 22nd Floor
Oakland California 94612-3550(US)

(72) Inventor: Casida, John Edward
1570 La Vereda Road
Berkeley California 94708(US)
Inventor: Elliott, Michael
9 Long Ridge Aston
Stevenage Hertfordshire(GB)
Inventor: Parkin, Donald
The Wellcome Foundation Limited
Berkhamsted
Hertfordshire(GB)

(74) Representative: Rollins, Anthony John et al
Group Patents & Agreements The Wellcome Research Laboratories Langley Court
Beckenham Kent BR3 3BS(GB)

## (54) Novel heterocyclic pesticidal compounds.

(57) Compounds of the formula (I)

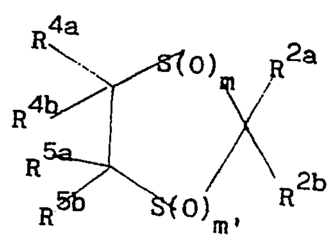

which contain between 10 and 27 carbon atoms, and wherein m and m' are independently selected from 0, 1 and 2; $R^{2a}$ is hydrogen, methyl, or ethyl; $R^{2b}$ is acetylene or contains between 3 and 18 carbon atoms and is a group $R^6$, wherein $R^6$ is a $C_{1-13}$ non-aromatic hydrocarbyl group, optionally substituted by a cyano or $C_{1-4}$ carbalkoxy group and/or by one or two hydroxy groups and/or by one to five halo atoms which are the same or different and/or by one to three groups $R^7$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^7$ and/or by a group -C≡CH, -C≡C-$R^6$ or C≡C-halo and/or by one to five halo atoms and/or by one to three $C_{1-4}$ haloalkyl groups wherein $R^6$ and $R^7$ are as hereinbefore defined; $R^{4b}$ and $R^{5b}$ are the same or different and are chosen from hydrogen, methyl or ethyl each optionally substituted by hydroxy, 1-5 fluoro atoms, methoxy, ethoxy, acetoxy, $C_{2-3}$ carbalkoxy or cyano or $R^{4b}$ and $R^{5b}$ and the carbon atoms of the dithiolane ring to which they are attached form a double bond; and $R^{4a}$ and $R^{5a}$ are the same or different and are each chosen from hydrogen or a $C_{1-11}$

EP 0 372 870 A2

non-aromatic hydrocarbyl group optionally substituted by 1 to 7 halo atoms, cyano, nitro, hydroxy, $C_{1-4}$ alkoxy or $C_{2-4}$ carbalkoxy groups or a phenyl or benzyl group optionally substituted by 1 to 5 halo atoms or $C_{1-4}$ haloalkyl groups, or $R^{4a}$ and $R^{5a}$ and the carbon atoms of the dithiolane ring to which they are attached form a cyclic or bridged polycyclic ring system containing between 3 and 9 atoms in the ring system and optionally substituted by a $C_{1-6}$ aliphatic group optionally substituted by 1 to 7 halo atoms or hydroxy or $C_{1-4}$ alkoxy groups; oxo, hydroxy a group $(O)_n$ $S(O)_r(O)_tR^8$ wherein $R^8$ is a $C_{1-4}$ aliphatic group optionally substituted by 1 to 5 halo atoms, n and t are each 0 or 1, r is 0, 1 or 2, the sum of n, r and t being between 0 and 3; $C_{1-4}$ alkoxy, $C_{1-4}$ carbalkoxy, one to 7 halo atoms, cyano, nitro or $C_{1-4}$ carbalkoxy provided that $R^{4a}$, $R^{4b}$, $R^{5a}$ and $R^{5b}$ are not all hydrogen and that one of $R^{4a}$, $R^{4b}$, $R^{5a}$ and $R^{5b}$ is not methyl when the others are hydrogen, are described as having activity as pesticides. Processes for their preparation, pesticidal formulations containing them and their use against pests are also disclosed.

# NOVEL HETEROCYCLIC PESTICIDAL COMPOUNDS

The present invention is concerned with a method of controlling pests such as arthropods, e.g. insects and acarine pests, and helminths, e.g. nematodes, by contacting the pests with novel pesticides. The invention is also concerned with the novel pesticides used for controlling the pests and processes for making such pesticides.

This invention was made with United States Government support under Grant No. P01 ES 00049 from the National Institutes of Health to The University of California. The United States Government has certain rights in this invention.

Current classes of pesticides effectively control some but not all pest species. It is also desirable to have new classes of pesticides since pests tend to develop resistance to any one pesticide, or sometimes to any one class of pesticide, after they have been selected with or exposed to such pesticides over a period of time.

US Patent No.2690988 discloses 1,3-dithiolanes having substituents at the 2- and the 4-positions having insecticidal activity.

It has been discovered that a further class of substituted dithiolanes has pesticidal activity.

Accordingly, the present invention provides a method for the control of pests which comprises administering to the pest or its environment an effective amount of a compound of the formula (I):

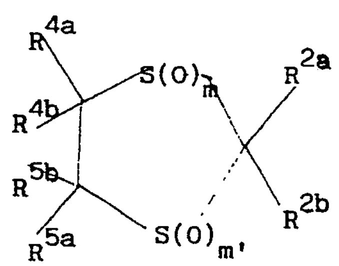

(I)

which contains between 10 and 27 carbon atoms, and wherein m and $m^1$ are independently selected from 0, 1 and 2; $R^{2a}$ a is hydrogen, methyl, or ethyl; $R^{2b}$ is acetylene or contains between 3 and 18 carbon atoms and is a group $R^6$, wherein $R^6$ is a $C_{1-13}$ non-aromatic hydrocarbyl group, optionally substituted by a cyano or $C_{1-4}$ carbalkoxy group and/or by one or two hydroxy groups and/or by one to five halo atoms which are the same or different and/or by one to three groups $R^7$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^7$ and/or by a group -C≡CH, -C≡C-$R^6$ or C≡C-halo and/or by one to five halo atoms and/or by one to three $C_{1-4}$ haloalkyl groups wherein $R^6$ and $R^7$ are as hereinbefore defined; $R^{4b}$ and $R^{5b}$ are the same or different and are chosen from hydrogen, methyl or ethyl each optionally substituted by hydroxy, 1-5 fluoro atoms, methoxy, ethoxy, acetoxy, $C_{2-3}$ carbalkoxy or cyano or $R^{4b}$ and $R^{5b}$ and the carbon atoms of the dithiolane ring to which they are attached form a double bond; and $R^{4a}$ and $R^{5a}$ are the same or different and are each chosen from hydrogen or a $C_{1-11}$ non-aromatic hydrocarbyl group optionally substituted by 1 to 7 halo atoms, cyano, nitro, hydroxy, $C_{1-4}$ alkoxy or $C_{2-4}$ carbalkoxy groups or a phenyl or benzyl group optionally substituted by 1 to 5 halo atoms or $C_{1-4}$ haloalkyl groups, or $R^{4a}$ and $R^{5a}$ and the carbon atoms of the dithiolane ring to which they are attached form a cyclic or bridged polycyclic ring system containing between 3 and 9 atoms in the ring system and optionally substituted by a $C_{1-6}$ aliphatic group optionally substituted by 1 to 7 halo atoms or hydroxy or $C_{1-4}$ alkoxy groups; oxo, hydroxy a group $(0)_n$ $S(0)_r(0)_tR^8$ wherein $R^8$ is a $C_{1-4}$ aliphatic group optionally substituted by 1 to 5 halo atoms, n and t are each 0 or 1, r is 0, 1 or 2, the sum of n, r and t being between 0 and 3; $C_{1-4}$ alkoxy, $C_{1-4}$ carbalkoxy, one to 7 halo atoms, cyano, nitro or $C_{1-4}$ carbalkoxy provided that $R^{4a}$, $R^{4b}$, $R^{5a}$ and $R^{5b}$ are not all hydrogen and that one of $R^{4a}$, $R^{4b}$, $R^{5a}$ and $R^{5b}$ is not methyl when the others are hydrogen.

By the term "halo" is meant fluoro, chloro, bromo or iodo.

By the term "non-aromatic hydrocarbyl" group is meant an alkyl, alkenyl or alkynyl group (including a cyclic alkyl or alkenyl group optionally substituted by alkyl, alkenyl or alkynyl; and alkyl or alkenyl substituted by cyclic alkyl and alkenyl).

By the term "6-membered aromatic ring" is meant phenyl and heteroaromatic rings such as pyridyl.

$R^{2b}$ suitably contains between 3 and 12 carbon atoms. $R^{2b}$ is suitably a $C_{3-9}$ alkyl, alkenyl or alkynyl group, each of which may be optionally substituted by halo or a group $R^7$, or a substituted phenyl or cyclohexyl group. The group $R^7$ is linked to the hydrocarbyl group or the aromatic ring via a hetero atom in $R^7$. Suitable substituents $R^7$ for the group $R^6$ include alkoxy, alkenyloxy, alkynyloxy, alkoxyalkoxy, acyloxy, alkynyloximino, trialkylsilyl, haloalkoxy, haloalkenyloxy, haloalkynyloxy, alkyloximino, mono or di-substituted alkylamino groups or a group $-(O)_nS(O)_r(O)_tR^8$ wherein $R^8$ is a $C_{1-4}$ aliphatic group optionally substituted by up to 5 halo atoms, n and t are each 0 or 1, r is 0, 1 or 2, the sum of n,r and t being between 0 and 3. When a silyl group is present this is normally adjacent to an ethynyl group. Preferred substituents $R^7$ include alkoxy, alkoxyalkoxy, alkenyloxy, alkynyloxy, haloalkoxy, haloalkenyloxy and haloalkynyloxy. Suitably $R^6$ is substituted by up to two substituents $R^7$ and preferably $R^6$ is unsubstituted or contains one substituent $R^7$. Preferably there is only one silyl group present. The sulphur atoms present may be in an oxidised form if desired. Preferably there is a maximum of two sulphur atoms present in $R^{2b}$. Suitably there is a maximum of four and preferably a maximum of three oxygen atoms in $R^{2b}$. Preferably there is only one nitrogen atom present in $R^{2b}$.

In one preferred embodiment, $R^{2b}$ is a phenyl group substituted at the 3- ,4- or 5-positions by one to three substituents each selected from halo, $C_{1-4}$ haloalkyl, $C_{1-4}$ haloalkoxy, $C_{1-4}$ haloalkylthio, cyano, or a group $(C{\equiv}C)_pR^9$ wherein p is 1 or 2 and $R^9$ is hydrogen, bromo, chloro, iodo or a group $S(O)_qR^{10}$ wherein q is 0, 1 or 2 and $R^{10}$ is trifluoromethyl, methyl or ethyl; or $R^9$ is an aliphatic group containing up to five carbon atoms optionally substituted by $C_{1-4}$ alkoxy, $C_{1-6}$ alkoxyalkoxy, $C_{1-8}$ acyloxy, halo or hydroxy or $R^9$ is a group $COR^{11}$ wherein $R^{11}$ is hydrogen, $C_{1-4}$ alkoxy, $C_{1-4}$ alkyl or a group $NR^{12}R^{13}$ wherein $R^{12}$ and $R^{13}$ are independently selected from hydrogen, methyl or ethyl; or $R^9$ is $SiR^{14}$, $R^{15}$, $R^{16}$ wherein $R^{14}$ and $R^{15}$ are the same or different and are each $C_{1-4}$ aliphatic groups and $R^{16}$ is a $C_{1-4}$ aliphatic group or phenyl provided that $R^{14}$, $R^{15}$ and $R^{16}$ do not contain more than 10 carbon atoms in total. The phenyl group is additionally optionally substituted at the 2- and/or 6-positions by fluoro or chloro. Suitably when the substituent is a group $(C{\equiv}C)_pR^9$, there is only one such substituent on the phenyl ring.

In a second preferred embodiment $R^{2b}$ is a group $-A(C{\equiv}C)Z$, wherein A is a $C_{3-5}$ aliphatic chain optionally containing a double bond and/or an oxygen atom and/or a group S(O)q wherein q is 0, 1 or 2 optionally substituted by halo, $C_{1-4}$ alkyl, $C_{1-4}$ haloalkyl, $C_{1-4}$ carbalkoxy or cyano and Z is hydrogen, $C_{1-5}$ alkyl, $C_{1-3}$ alkoxymethyl or a group $SiR^{14},R^{15},R^{16}$ wherein $R^{14}$, $R^{15}$ and $R^{16}$ are as hereinbefore defined.

In a third preferred embodiment $R^{2b}$ is a group $-BZ^1$, wherein B is a group $-CH_2O-$ or $CH_2S(O)q$ wherein q is 0, 1 or 2 or a $C_{2-3}$ aliphatic group each of which may be optionally substituted by one to three halo atoms and $Z^1$ is silyl substituted by three $C_{1-4}$ alkyl groups or $Z^1$ is a group

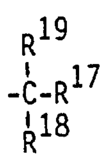

wherein $R^{17}$, $R^{18}$ and $R^{19}$ are the same or different and are each independently selected from halo, cyano, $C_{1-5}$ carbalkoxy, or a $C_{1-4}$ aliphatic group optionally substituted by halo,cyano, $C_{1-5}$ carbalkoxy, $C_{1-4}$ alkoxy or a group $S(O)_q$ $R^{20}$ wherein q is 0, 1 or 2 and $R^{20}$ is $C_{1-4}$ alkyl, or $R^{17}$, $R^{18}$ and $R^{19}$ are selected from $C_{1-4}$ alkoxy or a group $S(O)_wR^{21}$ wherein w is 0, 1 or 2 and $R^{21}$ is $C_{1-4}$ alkyl optionally substituted by fluoro or $R^{17}$ and $R^{18}$ are linked to form a $C_{3-6}$ cycloalkyl ring, or one of $R^{17}$, $R^{18}$ and $R^{19}$ may be hydrogen.

By the term "aliphatic group" is meant an alkyl, alkenyl or alkynyl group.

Most suitably B is a group $-C{\equiv}C-$ $-CH{\equiv}CH-$ or $-CH_2CH_2-$.

Preferably $Z^1$ is tertiary butyl, trichloromethyl or 2-methoxyprop-2-yl.

In a fourth preferred embodiment $R^{2b}$ is a group

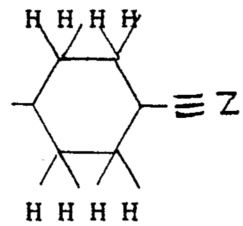

wherein Z is as hereinbefore defined:

Preferably $R^{2a}$ is hydrogen or methyl.

Suitably $R^{4b}$ and $R^{5b}$ are hydrogen or methyl. Preferably $R^{4b}$ and $R^{5b}$ are hydrogen.

Suitably at least one of $R^{4a}$ and $R^{5a}$ is hydrogen or methyl

Suitably $R^{4a}$ is hydrogen or a methyl, ethyl, isopropyl, isopropenyl, cyclopropyl, t-butyl or cyclobutyl group. Suitably $R^{5a}$ is a methyl, ethyl, isopropyl, isopropenyl, cyclopropyl, t-butyl or cyclobutyl group or $R^{4a}$ and $R^{5a}$ together with the carbon atoms of the dithiolane to which they are attached form a bridged ring system of the formula

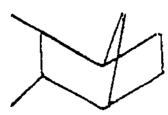

A preferred group of compounds of the formula (I) is that in which $R^{2b}$ contains a -(C≡C)- fragment or terminates in a group $Z^1$ as hereinbefore defined.

In a further aspect the present invention provides novel compounds comprising a compound of the formula (I) in which $R^{2b}$ contains a -(C≡C)-fragment or terminates in a group $Z^1$ as hereinbefore defined. Preferred substituents $R^{2a}$, $R^{4a}$, $R^{4b}$, $R^{5a}$ $R^{5b}$ , X and X′ for novel compounds of the formula (I) are as hereinbefore defined.

Some or the compounds of the formula (I) may exist in a number of stereoisomeric forms. The present invention encompasses both individual conformational and stereoisomers and mixtures thereof. The present invention also encompasses radiolabelled compounds of the formula (I), particularly those in which one carbon atom is $C^{14}$ or one or more hydrogen atoms are replaced by tritium.

The present invention also provides for the preparation of the compounds of the formula (I) by methods derived from those known in the art for the preparation of analogous compounds. Thus, the compounds may be prepared by (i) the reaction of a compound of the formula (II):

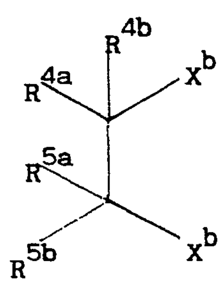

(II)

wherein $X^b$ is SH with a suitable aldehyde or ketone of the formula

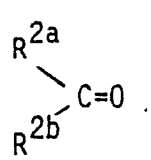

or a reactive derivative thereof, wherein $R^{2a}$, $R^{2b}$ $R^{4a}$, $R^{5a}$, $R^{4b}$ and $R^{5b}$ are as hereinbefore defined and, if

required, thereafter oxidizing one or both of the ring sulphur atoms.

The reaction is suitably carried out in the presence of a catalyst or of a dehydrating agent in a non-polar solvent at a non-extreme temperature. Suitable catalysts include a dimethyl formamide/dimethyl sulphate catalyst and catalysts such as sulphonic acids or perfluorinated resins thereof or Lewis acids such as boron trifluoride etherate, or stannic chloride or concentrated formic acid which also serves as the reaction medium. Suitable solvents include hydrocarbons such as benzene, toluene or xylene or chlorinated hydrocarbons such as dichloromethane. The reaction is normally performed between 0° and 200° and conveniently between 20° and 120°.

Suitable reactive derivatives of aldehydes and ketones include acetals and ketals.

The compounds of the formula (II) may be prepared from the corresponding diols wherein X is hydroxy via the sulphonate derivatives (i.e., compounds of the formula (II) wherein X is a group $OSO_2R^{22}$ wherein $R^{22}$ is $C_{1-4}$ alkyl or para-tolyl) as outlined in Appendix 1.

The aldehydes and ketones reacted with the dithiols of the formula (II) are either known in the literature or are prepared by literature methods, for example, European Publication 024 4 229.

(ii) When $R^{2a}$ is hydrogen, the reaction of a dithiaborinane-dimethylsulphide complex of a compound of the formula (II) with a carboxylic acid

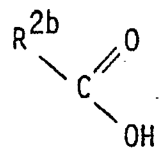

This reaction is carried out in the presence of a reducing agent such as stannous chloride in an inert solvent such as an ether, conveniently tetrahydrofuran, at a non-extreme temperature, for example between -20° and 100° and conveniently between 10° and 30°.

The dithiaborinane-dimethylsulphide complex is prepared from the corresponding dithiol by methods well known to those skilled in the art.

It is often convenient to prepare compounds of the formula (I) by interconversion from other compounds of the formula (I), for example:

(a)when it is required to prepare a compound of the formula (I) which contains an ethynyl group.

(i) by the reaction of the corresponding compound in which $R^{2b}$ is a 6-membered aromatic ring which contains iodo in place of $-C \equiv C-R^{36}$ with a compound $HC \equiv CR^6$ wherein $R^6$ is as hereinbefore defined. This reaction is carried out in the presence of a suitable palladium catalyst well known to those skilled in the art for this type of reaction, for example bistriphenylphosphine palladium dichloride, and a catalytic amount of a cuprous halide, such as cuprous iodide. The reaction will normally be carried out in the presence of basic solvent such as diethylamine or triethylamine at a non-extreme temperature, for example between -50° and 100° and conveniently at room temperature. The starting material, i.e. the iodophenyl dithiane may be prepared as described above.

(ii) By the conversion of a group, for example a group $CH=C(hal)_2$ or $(hal)CH=CH_2$ wherein hal is chloro or bromo, into an ethynyl group.

(b)when it is desired to prepare a compound of the formula (I) from a compound of formula I which contains a group $-C \equiv C-H$, by reaction of the anion from such a compound with an alkylating or acylating agent hal $R^6$, hal $R^9$ or hal Z respectively, wherein hal is halogen and $R^6$, $R^9$ or Z is other than hydrogen. This reaction is particularly suitable for the preparation of those compounds wherein $R^6$, $R^9$ or Z is a $C_{1-4}$ alkyl group or a group $COR^{23}$ wherein $R^{23}$ is a $C_{1-4}$ alkoxy group. The reaction is normally carried out in the presence of a strong base, such as an alkyllithium conveniently butyllithium in an inert solvent, such as an ether, for example tetrahydrofuran, at a non-extreme temperature, for example between -50° and 50°C and conveniently between -10° and 30°C. The starting material, e.g. the unsubstituted alkynylphenyl dithiane may be prepared as described above.

(c)when it is desired to prepare a compound of the formula (I) wherein $R^9$ or Z is hydrogen by the desilylation of a compound of the formula (I) wherein $R^9$ or Z is a tri-$C_{1-4}$ alkylsilyl group. This reaction may be carried out by methods well known to those skilled in the art, for example by reaction with tetrabutylammonium fluoride in an ether, such as tetrahydrofuran, at a non-extreme temperature, for example between 0° and 70°C and conveniently at room tempterature.

(d)The compounds of the formula (I) may contain two or more sulphur atoms which may be oxidised if required. Oxidations can be carried out by methods well known to those skilled in the art, for example

using peracids such as peracetic acid from hydrogen peroxide and acetic acid, or 3-chloroperbenzoic acid in chloroform or dichloromethane, or using periodate such as tetrabutylammonium periodate in a halogenated hydrocarbon, for example chloroform at a non-extreme temperature, for example between 0° and 100°C and conveniently between 10° and 30°C.

The compounds of formula (I) may be used to control pests such as arthropods, e.g. insect and acarine pests, and helminths, e.g. nematodes. Thus, the present invention provides a method for the control of arthropods and/or helminths which comprises administering to the arthropod and/or helminth or to their environment an effective amount of a compound of the formula (I). The present invention also provides a method for the control of arthropod and/or helminth infestations of animals (including humans) and/or of plants (including trees) and/or stored products which comprises administering an effective amount of a compound of the formula (I). The present invention further provides for the compounds of the formula (I) for use in human and veterinary medicine, in public health control and in agriculture for the control of arthropod and/or helminth pests.

By the term "control" is meant the amelioration in air, water, soil or foliage of present or future deleterious effects of pests and includes killing adults, larvae and eggs, the inhibition of reproduction, the repellency and/or knockdown of pests, and any other influence on behaviour.

Compounds of formula (I) are of particular value in the protection of field, forage, plantation, glasshouse, orchard and vineyard crops, of ornamentals and of plantation and forest trees, for example, cereals (such as maize, wheat, rice, millet, oats, barley, sorghum), cotton, tobacco, vegetables and salads (such as beans, cole crops, cucurbits, lettuce, onions, tomatoes and peppers), field crops (such as potato, sugar beet, ground nuts, soyabean, oil seed rape), sugar cane, grassland and forage crops (such as lucerne), plantations (such as of tea, coffee, cocoa, banana, oil palm, coconut, rubber, spices), orchards and groves (such as of stone and pip fruit, citrus fruits, kiwifruit, avocado, mango, olives and walnuts), vineyards, ornamental plants, flowers and shrubs under glass and in gardens and parks, forest trees (both deciduous and evergreen) in forests, plantations and nurseries and plants grown for industrial or pharmaceutical purposes (such as the evening primrose).

They are also valuable in the protection of timber (standing, felled, converted, stored or structural) from attack by sawflies (e.g. Urocerus) or beetles (e.g. scolytids, platypodids, lyctids, bostrychids, cerambycids, anobiids) termites (e.g.Isoptera) or other damaging pests.

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

Compounds of formula (I) are of value in the control of public health pests, for example cockroaches and ants.

Compounds of formula (I) are also of value in the control of arthropods or helminths which are injurious to, or spread or act as vectors of diseases in man and domestic animals, for example those hereinbefore mentioned, and more especially in the control of ticks, mites, lice, fleas, midges biting, nuisance and myiasis flies, mosquitos and hemiptrean bugs.

The compounds of Formula (I) may be used for such purposes by application of the compounds themselves or in diluted form in known fashion as a dip, spray, fog, lacquer, foam, dust, powder, aqueous suspension, paste, gel, cream, shampoo, grease, combustible solid, vapourising mat, combustible coil, bait, dietary supplement, wettable powder, granule, aerosol, emulsifiable concentrate, oil suspension, oil solution, pressure-pack, impregnated article, microcapsule, pour on formulation or other standard formulations well known to those skilled in the art. Sprays may be applied by hand or by means of a spray race or arch or by vehicle or aircraft mounted apparatus. The animal, soil, plant or other surface being treated may be saturated with the spray by means of high volume application or superficially coated with the spray by means of light or ultra low volume application. Dip concentrates are not applied per se, but diluted with water and the animals immersed in a dipping bath containing the dip wash. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material, such as that against which animals rub and transfer the material to their skins. Pour-on formulations are dispensed as a unit of liquid of small volume on to the backs of animals such that all or most of the liquid is retained on the animals.

Compounds of Formula (I) may be prepared either as formulations ready for use on the animals, plants or surface or as formulations requiring dilution prior to application, but both types of formulation comprise a

compound of Formula (I) in intimate admixture with one or more carriers or diluents. The carriers may be liquid, solid or gaseous or comprise mixtures of such substances, and the compound of Formula (I) may be present in a concentration of from 0.025 to 99% w/v depending upon whether the formulation requires further dilution.

Dusts, powders and granules and other solid formulations comprise the compound of formula (I) in intimate admixture with a powdered solid inert carrier for example suitable clays, kaolin, bentonite, attapulgite, adsorbent carbon black, talc, mica, silica, chalk, gypsum, tricalcium phosphate, powdered cork, magnesium silicate, vegetable carriers, starch or a diatomaceous earth. Such solid formulations are generally prepared by impregnating the solid diluents with solutions of the compound of formula (I) in volatile solvents, evaporating the solvents and, if desired, grinding the products so as to obtain powders and, if desired, granulating, compacting or encapsulating the products.

Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 99.5% w/v of the composition and may be selected from kerosene, ketones, alcohols, xylene, aromatic naphtha, water, mineral oil, aromatic and aliphatic esters, and other solvents known in the formulating art. The concentration of emulsifiers may be varied within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates, soaps, lecithins, hydrolysed glues, etc.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, or other solvents known in the art.

Wettable powders and emulsifiable concentrates will normally contain from 0.5 to 99.5% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of Formula (I) in intimate admixture with a dispersing agent and one or more surface active agents.

Aqueous suspensions of a compound of Formula (I) may comprise a suspension in water together with suspending, stabilizing or other agents. The suspensions or solutions may be applied per se or in a diluted form in known fashion.

Greases (or ointments) may be prepared from vegetable oils, synthetic esters of fatty acids or wool fat together with an inert base such as soft paraffin. A compound of Formula (I) is preferably distributed uniformly through the mixture in solution or suspension. Greases may also be made from emulsifiable concentrates by diluting them with an ointment base.

Pastes and shampoos are also semi-solid preparations in which a compound of Formula (I) may be present as an uniform dispersion in a suitable base such as soft or liquid paraffin or made on a non-greasy basis with glycerin, mucilage or a suitable soap. As greases, shampoos and pastes are usually applied without further dilution, they should contain the appropriate percentage of the compound of Formula (I) required for treatment.

Aerosol sprays may be prepared as a simple solution of the active ingredient in the aerosol propellant and co-solvent such as halogenated alkanes, propane, butane, dimethyl ether and the solvents referred to above, respectively. Pour-on formulations may be made as a solution or suspension of a compound of Formula (I) in a liquid medium. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body. Suitably the plastics material is polyvinyl chloride (PVC).

The concentration of the compound of formula (I) to be applied to an animal, premises, other substrates or outdoor areas will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited will vary according to the compound chosen, the method of application, area of application, concentration of the

compound in the applied formulation, factor by which the formulation is diluted and the nature of the formulation.

Undiluted formulations such as pour-on formulations in general will be applied at a concentration in the range from 0.1 to 20.0% w/w and preferably 0.1 to 10%. The amount of compound to be applied to stored products in general will lie in the range of from 0.1 to 20ppm. Space sprays may be applied to give an average initial concentration of 0.001 to 1 mg of compound of formula (I) per cubic metre of treated space.

Compounds of formula (I) are of use in the protection and treatment of plant species, in which case an effective insecticidal, acaricidal or nematocidal amount of the active ingredient is applied to the plant or the medium in which the plant is grown. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and 1kg/Ha. Typical formulations for agricultural use contain between 0.0001% and 50% of a compound of formula (I) and conveniently between 0.1 and 15% by weight of a compound of the formula (I).

Dusts, greases, pastes and aerosol formulations are usually applied in a random fashion as described above and concentrations of 0.001 to 20% w/v of a compound of Formula (I) in the applied formulation may be used.

The compounds of formula (I) have been found to have activity against the common housefly (Musca domestica). In addition, certain compounds of formula (I) have activity against other arthropod pests including Myzus persicae, Tetranychus urticae, Plutella xylostella, Culex spp. Tribolium castaneum, Sitophilus granarius, Periplaneta americana and Blattella germanica. The compounds of formula (I) are thus useful in the control of arthropods e.g. insects and acarines in any environment where these constitute pests, e.g. in agriculture, in animal husbandry, in public health control and in domestic situations.

Insect pests include members of the orders Coleoptera (e.g. Anobium,Ceutorhynchus,Rhynchophorus, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus or Anthrenus spp.), Lepidoptera (e.g. Ephestia, Mamestra, Earias, Pectinophora, Ostrinia, Trichoplusia, Pieris, Laphygma, Agrotis, Amathes, Wiseana, Tryporyza, Diatraea, Sporganothis, Cydia, Archips, Plutella, Chilo, Heliothis, Spodoptera or Tineola spp.), Diptera (e.g. Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops, Phytomyza, Ceratitis, Liriomyza and Melophagus spp.), Phthiraptera (Malophaga e.g. Damalina spp. and Anoplura e.g. Linognathus and Haematopinus spp.), Hemiptera (e.g. Aphis, Bemisia,Phorodon, Aeneolamia, Empoasca, Parkinsiella, Pyrilla, Aonidiella, Coccus, Pseudococcus, Helopeltis, Lygus, Dysdercus, Oxycarenus, Nezara, Aleurodes, Triatoma, Rhodnius, Psylla, Myzus, Meguora, Phylloxera, Adelyes, Niloparvata, Nephrotettix or Cimex spp.), Orthoptera (e.g. Locusta, Gryllus, Schistocerca or Acheta spp.), Dictyoptera (e.g. Blattella, Periplaneta or Blatta spp.), Hymenoptera (e.g. Athalia, Cephus, Atta Lasius, Solenopsis or Monomorium spp.), Isoptera (e.g. Odontotermes and Reticulitermes spp.), Siphonaptera (e.g. Ctenocephalides or Pulex spp.), Thysanura (e.g. Lepisma spp.), Dermaptera (e.g. Forficula spp.), Psocoptera (e.g. Peripsocus spp.) and Thysanoptera (e.g. Thrips tabaci),.

Acarine pests include ticks, e.g. members of the genera Boophilus,Ornithodorus, Rhipicephalus, Amblyomma, Hyalomma, Ixodes, Haemaphysalis, Dermacentor and Anocentor, and mites and manges such as Acarus, Tetranychus, Psoroptes, Notoednes, Sarcoptes, Psorergates, Chorioptes, Eutrombicula, Demodex, Panonychus, Bryobia and Eriophyes spp.

Nematodes which attack plants and trees of importance to agriculture, forestry, horticulture, either directly or by spreading bacterial, viral, mycoplasma or fungal diseases of the plants, include root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g.R. reniformis); Rotylenchus spp. (e.g. R. robustus); Helicotylenchus spp. (e.g. H. multicinctus); Hemicycliophora spp. (e.g. H. gracilis); Criconemoides spp. (e.g. C. similis); Trichodorus spp. (e.g. T primitivus); dagger nematodes such as Xiphinema spp. (e.g. X. diversicaudatum), Longidorus spp (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

Compounds of the invention may be combined with one or more other pesticidally active ingredients (for example pyrethroids, carbamates, lipid amides and organophosphates) and/or with attractants, repellents, bacteriocides, fungicides, anthelmintics and the like. Furthermore, the activity of compounds of the invention may be enhanced by the addition of a synergist or potentiator, for example: one of the oxidase

inhibitor class of synergists, such as piperonyl butoxide or propyl 2-propynylphenyl- phosphonate; a second compound of the invention; or a pyrethroid pesticidal compound. When an oxidase inhibitor synergist is present in a formulation of the invention, the ratio of synergist to compound of Formula (I) will be in the range 500:1-1:25 eg about 100:1 to 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin) and organic or inorganic bases e.g. trialkylamines such as triethylamine which can act as basic stabilisers and as scavengers.

The following Examples illustrate, in a non-limiting manner, preferred aspects of the invention. All temperatures are in degrees Celsius.

## EXPERIMENTAL

### General Synthetic Methods and Procedures:

Various compounds were synthesised and characterised in accordance with the following experimental procedures.

$^1$H N.M.R. spectra were obtained on a Bruker AM-250 or WM-300 spectrometer in deuterochloroform solutions with tetramethylsilane (TMS) as internal standard and are expressed as ppm from TMS, number of protons, number of peaks, coupling constant $J_{HZ}$.

Mass spectra were obtained on Finnigan 4500 or Hewlett Packard 5985B instruments. Gas-liquid chromatography (g.l.c.) was performed using a Pye Unicam GCD chromatograph fitted with a 3% OV210 column on Gas-Chrom Q and a flame-ionisation detector. Progress of reactions could also be conveniently monitored on plastic sheets (40 x 80 mm) precoated with 0.25mm layers of silica gel with fluorescent indicator and developed in benzene. Temperatures are in degrees Celsius throughout.

### Starting Materials

#### 1. Aldehydes and ketones

The following were commercially available :-

4-chlorobenzaldehyde, 4-bromobenzaldehyde,

4-[2-Trimethylsilyl)ethynyl]benzaldehyde was prepared as described by W.B.Austin, et al (J.Org.Chem., 46, 2280, 1981).

### Process 1

#### 4-Ethynylcyclohexanecarboxaldehyde

1) Di-isopropylamine (44.7ml) was dissolved in dry tetrahydrofuran (400ml) and cooled to -78° under nitrogen with mechanical stirring. A solution of n-butyllithium in hexane (1.6M. 197ml) was added. After stirring at -78° for 10 minutes a solution of dimethyl cyclohexane-1,4-dicarboxylate (52.6g) (Lancaster) in tetrahydrofuran (200ml) was added. After stirring for a further 30 minutes at -78° a solution of acetyl chloride (22.5ml) in tetrahydrofuran (200ml) was added. The reaction mixture was allowed to warm up to room temperature over a period of 3 hours. Water was then added and the mixture extracted with ether. The ethereal extracts were washed with water, saturated sodium bicarbonate solution, dilute hydrochloric acid and brine and were then dried over anhydrous magnesium sulphate. Evaporation under reduced pressure gave a colourless oil which was slowly distilled to yield dimethyl 1-acetylcyclohexane-1,4-dicarboxylate (23.3g. b.p. 114-120° /0.4mmHg).

Nuclear Magnetic Resonance spectrum (NMR) was as follows :

3.89,3H,s; 3.75,3H,s; 2.6-1.4,13H,m.

Infra-red spectrum (IR) (liquid film) 1740, 1710 $cm^{-1}$.

ii) Dimethyl 1-acetylcyclohexane-1-4-dicarboxylate (23.3g) was added to a solution of concentrated hydrochloric acid (253ml) in methanol (126ml). After refluxing for 10 hours the reaction mixture was poured into water and then extracted with dichloromethane. The organic phase was then washed with saturated sodium bicarbonate solution and brine. After drying over anhydrous magnesium sulphate the solvent was removed under reduced pressure to give methyl 4-acetylcyclohexanecarboxylate as a colourless oil. This was purified by distillation (b.p. 138-145°/l4mmHg).

Nuclear Magnetic Resonance spectrum (NMR) was as follows :

3.60,3H,s; 2.6-1.2,13H,m.

Infra-red spectrum (IR) (liquid film) 1730-1710$cm^{-1}$.

iii) Methyl 4-acetylcyclohexanecarboxylate (1.0g) in dry pyridine (0.7ml) was added to a stirred mixture of phosphorus pentachloride (2.45g) in dry pyridine (1.4ml). After stirring under reflux for 8 hours the reaction mixture was quenched by pouring into water. The mixture was then extracted with ether and the organic extracts washed with dilute hydrochloric acid, saturated sodium bicarbonate solution and brine. After drying over anhydrous magnesium sulphate, the solvent was removed under reduced pressure to give methyl 4-(1-chloroethenyl)cyclohexanecarboxylate as a pale yellow oil.

Nuclear Magnetic Resonance spectrum (NMR) was as follows :

5.03,2H,s; 3.62,3H,s; 2.80-1.09,10H,m.

Infra-red spectrum (IR) (liquid film). 1730$cm^{-1}$.

Mass spectrum (MS). M+1,203.

iv) Lithium aluminium hydride (283mg) was added to dry ether at 0° under a stream of nitrogen. After addition of methyl 4-(1-chloroethenyl) cyclohexanecarboxylate (1.0g), the reaction mixture was allowed to warm to 25° over a period of 2 hours. Sodium hydroxide solution (2.5ml 10%) was added cautiously. The ethereal solution was then decanted from the mixture, dried and evaporated to give 4-(1-chloroethynyl)-cyclohexylmethanol.

Nuclear Magnetic Resonance spectrum (NMR) was as follows :

4.97,2H,s; 3.6-3.25,2H,m; 2.20-0.8,10H,m.

Infrared spectrum (IR) (liquid film), 3400$cm^{-1}$.

v) n-Butyllithium (12ml,1.6M) was added at 0° under nitrogen to a stirred solution of 4-(1-chloroethenyl)cyclohexylmethanol (0.84g) in dry tetrahydrofuran (15ml). The reaction mixture was allowed to warm to room temperature and was stirred at 25° for 4 hours. Ice/water (100ml) was then added and the reaction mixture extracted with diethyl ether. After washing the organic extracts with brine and drying over anhydrous magnesium sulphate, the solvent was removed under reduced pressure. 4-Ethynylcyclohexylmethanol was purified by column chromatography on silica (eluted with ether:hexane; 2:3).

Nuclear Magnetic Resonance spectrum (NMR) was as follows:

3.32,2H,d; 2.80,1H,s; 2.29-0.80,11H,m.

Infra-red spectrum (IR) (liquid film) 3420, 3290$cm^{-1}$.

Mass spectrum (MS), M+1,139.

vi) Oxalyl chloride (354µl) was dissolved in dry dichloromethane (3ml) at -70° under nitrogen. Dimethyl sulphoxide (650µl) in dichloromethane (3ml) was then added. After stirring for 5 minutes a solution of 4-ethynylcyclohexylmethanol (0.5g) in dichloromethane (5ml) was added dropwise over 5 minutes. The reaction mixture was stirred for 30 minutes at -70° before triethylamine (2.5ml) was added. After warming to 25° over 3 hours, water was added and the organic phase separated, washed with dilute hydrochloric acid, saturated sodium bicarbonate solution and brine, and dried. Evaporation gave 4-ethynylcyclohexanecarboxaldehyde as a colourless oil.

Nuclear magnetic responance spectrum (NMR) was as follows :

9.61,1H,m; 3.0-1.0,9H,m.

Infra-red spectrum (IR) (liquid film) 3300, 2140, 1710$cm^{-1}$.

Hept-6-ynal

This was prepared using methodology described in stage vi) of Process 1 from hept-6-yn-1-ol (C.Crisan, Chem.Abs., 51, 5061b).

2. Dithols

The following were commercially available:-

Propane-1,2-dithol, butane-1,2-dithol, butane-2,3-dithiol.

2-Methylpropane-1,2-dithiol was prepared as described by M. Braid et al (J.Amer.Chem.Soc., 100 (19), 6160, 1978).

Pentane-2,3-dithiol was prepared by the method described by C.G.Overberger and A.Drucker (J.Org.Chem., 29,360,1964).

Cis-cyclohexane-1,2-dithiol was prepared as described by J. Houk and G.M. Whitesides (J.Amer.Chem. Soc., 109(22), 6825, 1987) from cis-cyclohexane -1,2-diol which was obtained from cyclohexene by the method described by M.F.Clark and L.N.Owen (J.Chem.Soc., 315,1949).

Bicyclo[2.2.1]hepta-exo-cis-2,3-dithiol was prepared by the method described by T.C.Shields and A.N.Kurts (J.Amer.Chem.Soc., 91, 5415, 1969).

Bicyclo[2.2.2]octa-cis-2,3-dithol,3-methylbicyclo[2.2.1]hepta-exo-cis-2,3-dithiol, 5-methylbicyclo[2.2.1]-hepta-exo-cis-2,3-dithiol and 5,6-dehydrobicyclo[2.2.1]hepta-exo-cis-2,3-dithiol were prepared in an analogous manner.

Process 2

Pentane-1,2-dithiol

i) Methanesulphonyl chloride (48.4g, 0.422moles) was added to pentane-1,2-diol (Aldrich, 20g, 0.192 moles) in dry dichloromethane (100ml) containing pyridine (50ml) at -10° at such a rate so as to maintain the temperature below -5°. The mixture was then allowed to warm to room temperature. After stirring for a further 1 hour. water was added and the two layers separated. The organic layer was washed with dilute (2N) hydrochloric acid, dried ($MgSO_4$), and the solvent removed to give an oil which solidified on standing. Dry ether was added and the solid collected to give 45g pentane-1,2-diol dimesylate.

$^1H$ nmr : 1.0 (3H,m,$CH_3$); 1.6 (4H,m,2$CH_2$); 3.1 (6H,s,); 4.2-4.9 (3H,m,CH-$CH_2$).

ii)Sodium trithiocarbonate [J.Org.Chem., 33(3), 1275,1968] (27.7ml, 2.4M in water, 0.068moles) was added to a solution of pentane-1,2-diol dimesylate (8g,0.0308moles) in dry dimethylformamide (100ml) and the mixture heated up to 100°. After 4 hours it was cooled and water (20ml) added followed by dilute (2N)-sulphuric acid (50ml) to give a thick yellow precipitate which slowly dissolved. The mixture was extracted with chloroform, dried ($MgSO_4$), and the solvent removed to give 4-n-propyl-1,3-dithiolane-2-thione as a red coloured oil (3.5g). This was used for the next stage without further purification.

$^1H$ nmr 0.9-2.0 (7H,m,n-Pr); 2.6-4.5 (3H,m,CH-$CH_2$).

iii) 4-n-Propyl-1,3-dithiolane-2-thione

The 2-thione (3.5g, 0.0197moles) in dry ether (10ml) was added to a suspension of lithium aluminium hydride (1.49g, 0.0197moles) in dry ether (180ml) under nitrogen at such a rate so as to maintain a gentle reflux. The mixture was then cooled and water (2ml) added followed by dilute (2N) hydrocloric acid (2ml) then water (2ml). The solid was filtered off, washed with ether, and the combined ether filtrates dried ($MgSO_4$). The solvent was removed to give pentane-1,2-dithiol as a yellow liquid (2.21g).

$^1H$ nmr : 0.8-1.1 (3H,m,Me); 1.3-1.8 (6H,m,2$CH_2$ + 2SH); 2.6-3.0 (3H,m,$CH_2$S + CHS).

Process 3

3,3-Dimethylbutane-1,2-dithiol

3,3-Dimethylbutane-1,2-diol.dimesylate (obtained from the diol [Aldrich] by the method shown in Process 2(1)) (8g) was refluxed with sodium sulphide (8.4g) and sulphur (1.03g) in dry dimethylformamide (100ml) for 3hours. The mixture was cooled and poured onto ice/water (200ml) and extracted with diethyl ether. The aqueous layer was made acidic with concentrated hydrochloric acid and reextracted with diethyl ether. The combined ether layers were washed with water, dried ($MgSO_4$) and the ether removed to give an amber oil (4.3g). The oil was taken up in diethyl ether (10ml) and added to a suspension of lithium

aluminium hydride (1.1g) in dry ether (100ml) at such a rate so as to maintain a steady reflux. After stirring for a further 1 hour, water (2ml) was carefully added followed by dilute sulphuric acid (2ml), then more water (2ml). The solid was filtered off and the solvent removed to give a pale yellow liquid (2.7g) consisting mainly of 3,3-dimethylbutane-1,2-dithiol. The oil was used without further purification.

Preparation of Dithiolanes

Method 1

2-(4-Bromophenyl)-4,5-dimethyl-1,3-dithiolane

2,3-Butanedithiol (100mg) was added to a solution of 4-bromobenzaldehyde (0.15g) in formic acid (96%, 1ml). After 18 hours at room temperature, the solvent was separated from the product, which was dissolved in dichloromethane (2ml), washed with saturated sodium hydrogen carbonate solution and dried ($MgSO_4$). After the solvent had been evaporated in nitrogen, the product (0.23g) crystallised on standing at room temperature and was characterised by nmr and GC-MS. Recrystallisation gave white needles, m.p. 34°, a mixture of 2 isomers.

2-(4-Ethynylphenyl )-4,5-dimethyl-1,3-dithiolane

By a similar procedure, 4-ethynylbenzaldehyde (193 mg) with 2,3-butanedithiol (0.18g) gave a yellow oil (318 mg) which was characterised by nmr and shown by GC-MS to contain only 2 compounds.

Method 2

2-(Hex-5-ynyl )-3a,4,5,6,7,7a-hexahydro-4,7-methano-(2H)-1,3-benzodithiole

A solution of bicyclo[2.2.1] hepta-exo-cis-2,3-dithiol (0.4g) (ref. T.C. Shields et al J.Amer.Chem.Soc., 1969, 91, 5415) and hept-6-yn-1-al (0.28g) in dry toluene (50ml) containing p-toluenesulphonic acid (50mg) was heated at reflux in a Dean and Stark apparatus for 1 hour. After cooling, water and diethyl ether were added and the solutions separated. The ethereal solution was dried over magnesium sulphate and evaporated in vacuo. Chromatography on silica gel, eluting with diethyl ether:hexane; 1:9 gave 2-(hex-5-ynyl)- 3a,4,5,6,7,7a-hexahydro-4,7-methano-(2H)-1,3-benzodithiole (100mg).

Method 3

2-(4-Ethynylphenyl)-3a,4,5,6,7,7a-hexahydro-4,7-methano-(2H)-1,3-benzodithiole

A solution of tetra-n-butylammonium fluoride in tetrahydrofuran (1M,5.25ml) was added dropwise to a stirred solution of 2-(4-trimethylsilylethynylphenyl)-3a,4,5,6,7,7a-hexahydro-4,7-methano-(2H)-1,3-benzodithiole (1.5g) kept under nitrogen. After 4 hours water and diethyl ether were added and the solutions separated. The organic phase was dried over magnesium sulphate and evaporated in vacuo. Crystallisation from hexane yielded 2-(4-ethynylphenyl)-3a,4,5,6,7,7a-hexahydro-4,7-methano-(2H)-1,3-benzodithiole.

BIOLOGICAL ACTIVITIES

Spray Tests

The activity of the compounds of the invention were tested by dissolving the compounds in acetone (5%) and then diluting in water: "Symperonic" (94.5% : 0.5%) to give a water emulsion. The solution was then used to treat the following insects.

Musca domestica:

20 female Musca were contained in a cardboard cylinder with gauze over either end. Solution containing the compound was sprayed onto the insects so enclosed and mortality assessed after 48 hours at 25°C.

The following compound was active on M.domestica at less than 1000ppm.

4,5,6,13,14,15,16,17,22,23,28,29,30

Plutella xylostella:

7 Plutella larvae (3rd instar) were sprayed with the solution containing the compound and added to a Chinese cabbage leaf which had been similarly sprayed and left to dry. Mortality was assessed after 2 days at 25°C.

The following compound was active on P.xylostella at less than 1000ppm.

16,18,19,29,30

<Myzus persicae:

10 adult Myzus were placed on a leaf disc of chinese cabbage. 24 hours later the disc was sprayed with the solution containing the compound. Mortality is assessed after 2 days at 25°C.

The following compound was active on M.persicae at less than 1000ppm.

2,6,15,16,17,23,30

Diabrotica undecimpunctata

Filter paper was sprayed with the solution containing the compound. Seven 2nd instar larvae were then added to the filter paper, together with a cube of artificial diet. Activity was assessed after 48 hours.

The following compound was active on D.undecimpunctata at less than 1000ppm.

5,6,9,13,17,28,29

Topical Application Tests

Lethal Activity Against Blatella germanica

The activity of compounds of the invention against anaesthetised male Blattella germanica (WRL strain) was demonstrated by the topical application to the test insect of a solution of the compound under test in butanone. Mortality was assessed after 6 days.

The following compounds were active at less than 5µg/insect

4,5,6,9,17,28,29,30

The activity of compounds of the invention against unanaethetised female Musca domestica was demonstrated by the topical application to the test insect of a solution of the compound under test in butanone. The compounds were applied with the synergist piperonyl butoxide. Mortality was assessed at 48 hours.

The following compounds were active at < 1µg/insect

5,6

Formulations

| 1. Emulsifiable Concentrate | |
|---|---|
| Compound of formula (I) | 10.00 |
| Alkyl phenol ethoxylate* | 7.50 |
| Alkyl aryl sulphonate* | 2.50 |
| $C_{8-13}$ aromatic solvent | 80.00 |
| | 100.00 |

* = Surfactant

| 2. Emulsifiable Concentrate | |
|---|---|
| Compound of formula (I) | 10.00 |
| Alkyl phenol ethoxylate* | 2.50 |
| Alkyl aryl sulphonate* | 2.50 |
| Ketonic solvent | 64.00 |
| $C_{8-13}$ aromatic solvent | 18.00 |
| Antioxidant | 3.00 |
| | 100.00 |

* = Surfactant

| 3. Wettable Powder | |
|---|---|
| Compound of formula (I) | 5.00 |
| $C_{8-13}$ aromatic solvent | 7.00 |
| $C_{18}$ aromatic solvent | 28.00 |
| China clay | 10.00 |
| Alkyl aryl sulphonate* | 1.00 |
| Naphthalene sulphonic acid* | 3.00 |
| Diatomaceous earth | 46.00 |
| | 100.00 |

* = Surfactant

| 4. Dust | |
|---|---|
| Compound of formula (I) | 0.50 |
| Talc | 99.50 |
| | 100.00 |

| 5. Bait | |
|---|---|
| Compound of formula (I) | 0.5 |
| Sugar | 79.5 |
| Paraffin wax | 20.0 |
| | 100.00 |

| 6. Emulsion Concentrate | |
|---|---|
| Compound of formula (I) | 5.00 |
| $C_{8-13}$ aromatic solvent | 32.00 |
| Cetyl alcohol | 3.00 |
| Polyoxyethylene glycerol monooleate* | 0.75 |
| Polyoxyethylene sorbitan esters* | 0.25 |
| Silicone solution | 0.1 |
| Water | 58.9 |
| | 100.00 |

* = Surfactant

| 7. Suspension Concentrate | |
|---|---|
| Compound of formula (I) | 10.00 |
| Alkyl aryl ethoxylate* | 3.00 |
| Silicone solution | 0.1 |
| Alkane diol | 5.0 |
| Fumed silica | 0.50 |
| Xanthan gum | 0.20 |
| Water | 80.0 |
| Buffering agent | 1.2 |
| | 100.00 |

* = Surfactant

| 8. Microemulsion | |
|---|---|
| Compound of formula (I) | 10.00 |
| Polyoxyethylene glycerol monooleate* | 10.00 |
| Alkane diol | 4.00 |
| Water | 76.00 |
| | 100.00 |

* = Surfactant

| 9. Water Dispersible Granules | |
|---|---|
| Compound of formula (I) | 70.00 |
| Polyvinyl pyrrolidine | 2.50 |
| Alkyl aryl ethoxylate | 1.25 |
| Alkyl aryl sulphonate | 1.25 |
| China clay | 25.00 |
| | 100.00 |

| 10. Granules | |
|---|---|
| Compound of formula (I) | 2.00 |
| Alkyl phenol ethoxylate* | 5.00 |
| Alkyl aryl sulphonate* | 3.00 |
| $C_{8-13}$ aromatic solvent | 20.00 |
| Kieselguhr granules | 70.00 |
| | 100.00 |

* = Surfactant

| 11. Aerosol (pressure pack) | |
|---|---|
| Compound of formula (I) | 0.3 |
| Piperonyl butoxide | 1.5 |
| $C_{8-13}$ saturated hydrocarbon solvent | 58.2 |
| Butane | 40.0 |
| | 100.00 |

| 12. Aerosol (pressure pack) | |
|---|---|
| Compound of formula (I) | 0.3 |
| $C_{8-13}$ saturated hydrocarbon solvent | 10.0 |
| Sorbitan monooleate* | 1.0 |
| Water | 40.0 |
| Butane | 48.7 |
| | 100.00 |

* = Surfactant

| 13. Aerosol (pressure pack) | |
|---|---|
| Compound of formula (I) | 1.00 |
| $CO_2$ | 3.00 |
| Polyoxyethylene glycerol monooleate* | 1.40 |
| Propanone | 38.00 |
| Water | 56.60 |
| | 100.00 |

* = Surfactant

| 14. Lacquer | |
|---|---|
| Compound of formula (I) | 2.50 |
| Resin | 5.00 |
| Antioxidant | 0.50 |
| High aromatic white spirit | 92.0 |
| | 100.00 |

| 15. Spray (ready to use) | |
|---|---|
| Compound of formula (I) | 0.10 |
| Antioxidant | 0.10 |
| Odourless kerosene | 99.8 |
| | 100.00 |

| 16. Potentiated Spray (ready to use) | |
|---|---|
| Compound of formula (I) | 0.10 |
| Piperonyl butoxide | 0.50 |
| Antioxidant | 0.10 |
| Odourless kerosene | 99.30 |
| | 100.00 |

| 17. (Microencapsulated) | |
|---|---|
| Compound of formula (I) | 10.0 |
| $C_{8-13}$ aromatic solvent | 10.0 |
| Aromatic di-isocyanate# | 4.5 |
| Alkyl phenol ethoxylate* | 6.0 |
| Alkyl diamine# | 1.0 |
| Diethylene triamine | 1.0 |
| Concentrated hydrochloric acid | 2.2 |
| Xanthan gum | 0.2 |
| Fumed silica | 0.5 |
| Water | 64.6 |
| | 100.00 |

* = Surfactant
# = react to form the polyurea walls of the microcapsule
Antioxidant could be any of the following individually or combined
Butylated hydroxytoluene
Butylated hydroxyanisole
Vitamin C (ascrobic acid)

The following Examples illustrate, in a non-limiting manner, preferred aspects of the invention.

## EXPERIMENTAL

### General Synthetic Methods and Procedures:

Various compounds were synthesised and characterised in accordance with the following experimental procedures.

$^1H$ N.m.r. spectra were obtained on a Bruker AM-250 spectrometer in deuterochloroform solutions with

tetramethylsilane as internal standard and are expressed as ppm from TMS, number of protons, number of peaks, coupling constant J Hz.

Progress of reactions could also be conveniently monitored on Aluminium sheets (40 x 80 mm) precoated with 0.25 mm layers of silica gel with fluorescent indicator and developed in appropriate solvent or solvent mixture. Temperatures are in degrees Celsius throughout.

Conventional work up was performed as follows:

The reaction mixture was partitioned between an organic solvent and water. The phases were separated and the organic phase washed with at least an equivalent volume of dilute aqueous base as appropriate, and then with a saturated brine wash. The organic phase was then dried over a drying agent, suitably magnesium sulphate, and filtered. The volatile solvents were removed and the resulting product subjected to the appropriate purification and used in the next stage of synthesis or analysed as the final product.

The aldehyde, cinnamic acid and amine starting materials were obtained from Aldrich, BDH, Fluorochem, Fluka or Lancaster Synthesis with the exception of the following whose preparation is described below.

Appendix 1

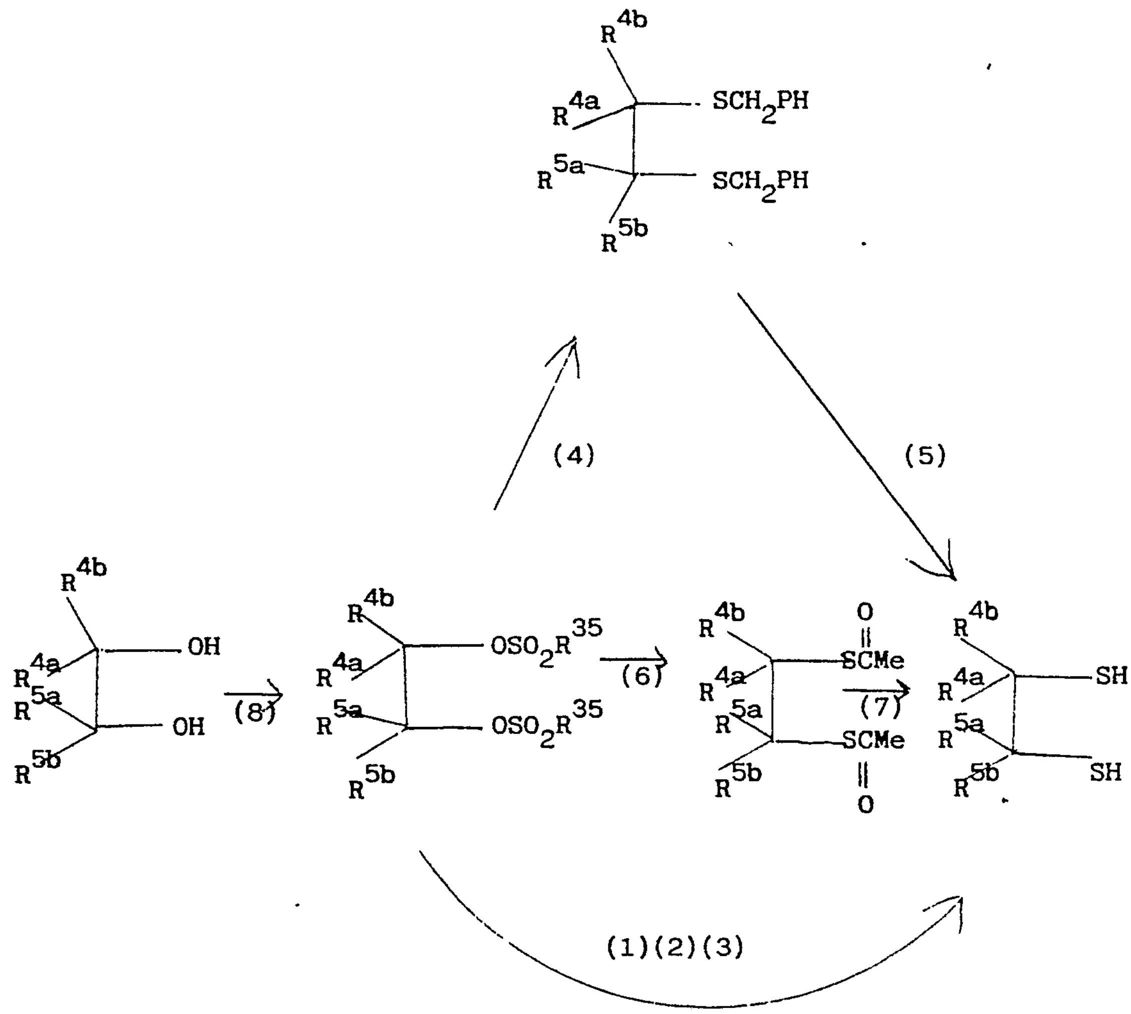


(1)$Na_2CS_3$,$H_2O$ (2) HCl (3) $LiAlH_4$, $Et_2O$ (4) $NaSCH_2Ph$, DMF
(5) Na,liq.$NH_3$ (6) KSC(O).Me,EtOH (7) $H_2NCH_2CH_2NH_2$,EtOH
(8) $R^{35}SO_2Cl$,pyridine

TABLE 1A

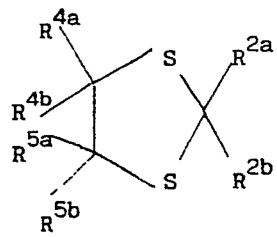

| Compound Number | $R^{2a}$ | $R^{2b}$ | $R^{4a}$ | $R^{4b}$ | $R^{5a}$ | $R^{5b}$ | Isomer Ratio Trans;Cis |
|---|---|---|---|---|---|---|---|
| 1 | H | 4-Bromophenyl | H | Me | H | Me | 2 Isomers |
| 2 | H | 4-Ethynylphenyl | H | Me | H | Me | 1 Isomers |
| 3 | H | 4-Trimethylsilyl-ethynylphenyl | H | Me | H | H | 1:1 |
| 4 | H | 4-Ethynylphenyl | H | Me | H | H | 1:1 |
| 5 | H | 4-Trimethylsilyl-ethynylphenyl | H | Et | H | H | 1:1 |
| 6 | H | 4-Ethynylphenyl | H | Et | H | H | 1:1 |
| 7 | H | 4-Trimethylsilyl-ethynylphenyl | Me | Me | H | H | - |
| 8 | H | 4-Ethynylphenyl | Me | Me | H | H | - |
| 9 | H | 4-Ethynylphenyl | H | Et | H | Me | 4 Isomers |
| 10 | H | 4-Trimethylsilyl-ethynylphenyl | H | Et | H | Me | 4 Isomers |
| 11 | H | 4-Trimethylsilyl-ethynylphenyl | H | $-(CH_2)_4-$ | | H | |
| 13 | H | 5-Hexynyl | H | Et | H | H | 1:1 |
| 14 | H | 4-Trimethylsilyl-ethynylphenyl | H | nPr | H | H | 1:1 |
| 15 | H | 4-Ethynylphenyl | H | nPr | H | H | 1:1 |

TABLE 1b

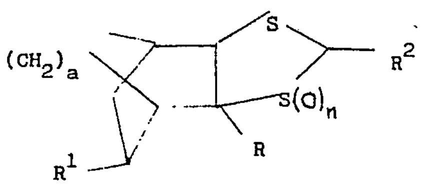

| Compound Number | $R^2$ | a | n | R | $R^1$ | Isomer Ratio a : b |
|---|---|---|---|---|---|---|
| 16 | 5-Hexynyl | 1 | 0 | H | H | a |
| 17 | 4-Ethynylphenyl | 1 | 0 | H | H | a |
| 18 | 4-Ethynylphenyl | 1 | 0 | H | H | b |
| 19 | 3,3-Dimethylbut-1-ynyl | 1 | 0 | H | H | 2:1 |
| 20 | 4-Trimethylsilylethynylphenyl | 2 | 0 | H | H | a |
| 21 | 4-Ethynylphenyl | 2 | 0 | H | H | a |
| 22 | 5-Hexynyl | 2 | 0 | H | H | a |
| 23 | 5-Hexynyl | 1 | 1 | H | H | a |
| 24 | 4-Ethynylphenyl | 1 | 1 | H | H | a |
| 25 | 4-Trimethylsilylethynylphenyl | 1 | 0 | Me | H | a |
| 26 | 4-Ethynylphenyl | 1 | 0 | Me | H | 4:1 |
| 27 | 4-Trimethylsilylethynylphenyl | 1 | 0 | H | Me | a (isomers) |
| 28 | 4-Ethynylphenyl | 1 | 0 | H | Me | 1:1 (isomers) |
| 29 | trans-4-Ethynylcyclohexyl | 1 | 0 | H | H | 95:5 |

TABLE 1c

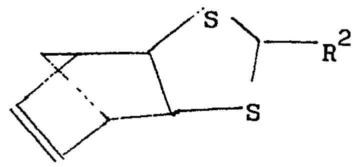

| Compound No. | $R^2$ |
|---|---|
| 30 | 5-Hexynyl |

TABLE 2A

Nuclear Magnetic Resonance Spectra:- H,$CDCl_3$ and expressed as p.p.m. downfield from TMS (number of protons, multiplicity, assignment).

1. 1.4(6H,m); 3.4-4.0(2H,m); 5.6(1H,m); 7.4(4H,m).

3. 0.2(9H,s,$SiMe_3$); 1.45(3H,2d,Me); 3.0-4.1(3H,m,$CH_2$CH); 5.60 and 5.65 (1H,2s,2-CH); 7.35-7.50-(4H,m,Ar-H).

4. 1.45(3H,2d,Me); 3.0-4.1(3H,m,$CH_2$-CH); 5.60 and 5.65(1H,2s,2-CH); 7.35-7.5(4H,m,Ar-H).

5. 0.2(9H,s.$SiMe_3$); 1.05(3H,m,Me); 1.7-1.9(2H,m,$CH_2$); 3.0-3.9(3H,m, $CH_2$CH); 5.55(1H,s,2-CH); 7.3-7.45(4H,m,Ar-H).

6. 1.0(3H,m,Me); 1.7-1.9(2H,m,$CH_2$); 3.0-3.9(3H,m,$CH_2$CH); 5.60(1H, s,2-CH); 7.35-7.50(4H,m,Ar-H).

7. 0.2(9H,s,$SiMe_3$); 1.6(6H,2s,2Me); 3.08-3.25(2H,m,$CH_2$); 5.68(1H,s, 2-CH); 7.35-7.50(4H,m,Ar-H).

8. 1.65(6H,2s,2Me); 3.05-3.25(3H,m,$CH_2$ + CH); 5.68(1H,s,2-CH); 7.35-7.50(4H,m,Ar-H).

9. 1.05(3H,m,Me); 1.4-2.0(5H,m,$CH_2$ + Me); 3.05(1H,s,CH); 3.3-3.95(2H,m,2CH); 5.55-5.60(1H,4s,2-CH); 7.3-7.5(4H,m,Ar-H).

10. 0.25(9H,s,$SiMe_3$); 1.05(3H,m,Me); 1.4-2.0(5H,m,$CH_2$ + Me); 3.3-3.9(2H,m,2CH); 5.55-5.60(1H,4s,2-CH); 7.35-7.50(4H,m,Ar-H).

11. 0.2(9H,s,$SiMe_3$); 1.4-2.25(8H,m,cyclohexyl $CH_2$); 3.8 and 4.0(2H,m,CH-S); 5.70(1H,s,2-CH); 7.35-7.50(4H,m,Ar-H).

13. 1.0(3H,m,Me); 1.4-2.2(11H,m,5CH2 + CCH); 2.8-3.6(3H,m,$CH_2$-S + CH-S); 4.4(1H,m,2-CH).

14. 0.2(9H,s,$SiMe_3$); 1.0(3H,m,Me); 1.45(4H,m); 3.0-4.1(3H,m,$CH_2$CH); 5.60 and 5.65(1H,2s,2-CH); 7.35-7.50(4H,m,Ar-H).

15 1.0(3H,m,Me); 1.6(4H,m); 3.0-4.1(3H,m); 5.6(1H,s); 7.4(4H,m).

TABLE 2B

Nuclear Magnetic Resonance Spectra:- H, $CDCl_3$ and expressed as p.p.m. downfield from TMS (number of protons, mulitplicity, assignment).

16. 1.1-2.3(17H,m,$CH_2$ + CH); 3.5(2H,m,CH-S); 4.3(1H,t,CH).

17. 1.0-2.5(8H,m,$CH_2$ + CH); 3.0(1H,s,CCH); 3.6 and 3.8(2H,m,CH-S); 5.4(1H,s,CH); 7.3(4H,m,Ar-H).

18. 1.02.5(8H,m,$CH_2$ + CH); 3.0(1H,s,≡CH); 3.6 and 3.8(2H,m,CH-S); 5.6(1H,s,CH); 7.3(4H,m,Ar-H).

19. 1.2-2.4(8H,m,$CH_2$ + CH); 1.2(9H,s,$CMe_3$); 3.6 and 3.8(2H,m,CH-S); 5.0 and 5.1(1H,s,CH).

20. 0.2(9H,s,$SiMe_3$); 1.3-2.3(10H,m,$CH_2$ + CH); 3.9(2H,m,CH-S); 5.45(1H,s,CH); 7.35-7.55(4H,m,Ar-H).

21. 1.3-2.3(10H,m,$CH_2$ + CH); 3.10(1H,s,≡CH); 4.4(2H,m,CH-S); 5.50(1H,s,CH); 7.35-7.50(4H,m,Ar-H).
22. 1.2-2.2(19H,m,$CH_2$ + CH); 3.8(2H,m,CH-S); 4.4(1H,t,CH).
23. 1.1-2.9(17H,m,$CH_2$ + CH); 3.5(2H,m,CH); 3.85(1H,m,CH).
24. 1.2-2.3(8H,m,$CH_2$ + CH); 3.1(1H,s,CH); 2.9 and 3.6(2H,m,CH-S); 4.9(1H,s,CH) 7.4(4H,s,Ar-H).
25. 0.2(9H,s,$SiMe_3$); 1.0-2.8(8H,m,$CH_2$ + CH); 1.55(3H,s,Me); 3.1(1H,m,CH-S); 5.45(1H,s,CH); 7.3-7.5-(4H,m,Ar-H).
26. 1.0-2.8(8H,m,$CH_2$ + CH); 1.55(3H,s,Me); 3.05(1H,s,CH); 3.1 and 3.2(1H,m,CH-S); 5.45 and 5.75-(1H,s,CH); 7.3-7.5(4H,m,Ar-H).
27. 0.2(9H,s,$SiMe_3$); 0.6-2.6(7H,m,$CH_2$ + CH); 1.0(3H,2s,Me);3.6 and 4.1(2H,m,CH-S); 5.45(1H,s,CH); 7.35-7.55(4H,m,Ar-H).
28. 0.6-2.6(7H,m,$CH_2$ + CH); 1.0(3H,m,Me); 3.10(1H,m,≡CH); 3.6-4.2(2H,m,CH-S); 5.45-5.70-(1H,m,CH); 7.4-7.6(8H,m,Ar-H).
29, 1.0-2.2(19H,m,CH2 + CH + CCH); 3.5(2H,m,CH-S); 4.2 and 4.3(1H,d,CH).

TABLE 1c

Nuclear Magnetic Resonance Spectra:- H, $CDCl_3$ and expressed as p.p.m. downfield from TMS (number of protons, multiplicity, assignment).

30. 1.4-2.8(13H,m,$CH_2$ + CH + CCH); 3.60(2H,m,CH-S); 4.75(1H,t,CH); 6.2(2H,m,CH = CH);

TABLE 3A

| Compound Number | Mass Spectrum Chemical Ionisation M+1 | Mp°C or $N_D$ | Description | Method of Preparation |
|---|---|---|---|---|
| 1 | 289 | 34 | white solid | 1′ |
| 2 | 235 | - | yellow oil | 1 |
| 3 | 293 | 48 | pale yellow solid | 2 |
| 4 | 227 | 1.637 | yellow oil | 3 |
| 5 | 307 | 1.588 | pale yellow solid | 2 |
| 6 | 235 | 1.621 | pale yellow oil | 3 |
| 7 | 307 | 72 | white solid | 2 |
| 8 | 235 | 46 | yellow solid | 3 |
| 9 | 249 | 1.599 | yellow oil | 3 |
| 10 | 321 | - | pale yellow solid | 2 |
| 11 | 333 | 101.8 | yellow solid | 2 |
| 12 | | | | |
| 13 | 215 | 1.532 | pale yellow oil | 2 |
| 14 | 321 | - | colourless oil | 2 |
| 15 | 249 | - | yellow oil. | 3 |

TABLE 3B

| Compound Number | Mass spectrum Chemical Ionisation M+1 | Mp°C or $N_D$ | Description | Method of Preparation |
|---|---|---|---|---|
| 16 | 253 | 1.562 | pale yellow oil | 2 |
| 17 | 273 | 85 | pale yellow solid | 3 |
| 18 | 273 | 150 | pale yellow solid | 3 |
| 19 | 253 | 1.330 | yellow oil | 2 |
| 20 | 359 | 153 | white solid | 2 |
| 21 | 287 | 158 | white solid | 3 |
| 22 | 267 | 38 | white solid | 2 |
| 23 | 269 | 62 | white solid | 2 |
| 24 | 289 | DECOMP 270 | white solid | 3 |
| 25 | 359 | 127.7 | white solid | 2 |
| 26 | 287 | 106.4 | white solid | 3 |
| 27 | 359 | 97.5 | white solid | 2 |
| 28 | 287 | 81.4 | solid | 3 |
| 29 | 279 | 113.6 | white solid | 2 |

TABLE 3C

| Compound Number | Mass spectrum Chemical Ionisation M+1 | Mp°C or $N_D$ | Description | Method of Preparation |
|---|---|---|---|---|
| 30 | 251 | 1.562 | pale yellow oil | 2 |

## Claims

1) A compound of the formula (I)

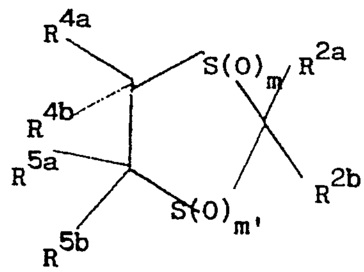

which contains between 10 and 27 carbon atoms, and wherein m and m' are independently selected from 0, 1 and 2; $R^{2a}$ is hydrogen, methyl, or ethyl; $R^{2b}$ is acetylene or contains between 3 and 18 carbon atoms and is a group $R^6$, wherein $R^6$ is a $C_{1-13}$ non-aromatic hydrocarbyl group, optionally substituted by a cyano or $C_{1-4}$ carbalkoxy group and/or by one or two hydroxy groups and/or by one to five halo atoms which are the same or different and/or by one to three groups $R^7$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^7$ and/or by a group -C≡CH, -C≡C-$R^6$ or C≡C-halo and/or by one to five halo atoms and/or by one to three $C_{1-4}$ haloalkyl groups wherein $R^6$ and $R^7$ are as hereinbefore defined; $R^{4b}$ and $R^{5b}$ are the same or different and are chosen from hydrogen, methyl or ethyl each optionally substituted by hydroxy, 1-5 fluoro atoms, methoxy, ethoxy, acetoxy, $C_{2-3}$ carbalkoxy or cyano or $R^{4b}$ and $R^{5b}$ and the carbon atoms of the dithiolane ring to which they are attached form a double bond; and $R^{4a}$ and $R^{5a}$ are the same or different and are each chosen from hydrogen or a $C_{1-11}$ non-aromatic hydrocarbyl group optionally substituted by 1 to 7 halo atoms, cyano, nitro, hydroxy, $C_{1-4}$ alkoxy or $C_{2-4}$ carbalkoxy groups or a phenyl or benzyl group optionally substituted by 1 to 5 halo atoms or $C_{1-4}$ haloalkyl groups, or $R^{4a}$ and $R^{5a}$ and the carbon atoms of the dithiolane ring to which they are attached form a cyclic or bridged polycyclic ring system containing between 3 and 9 atoms in the ring system and optionally substituted by a $C_{1-6}$ aliphatic group optionally substituted by 1 to 7 halo atoms or hydroxy or $C_{1-4}$ alkoxy groups; oxo, hydroxy a group $(O)_n$ $S(O)_r(O)_tR^8$ wherein $R^8$ is a $C_{1-4}$ aliphatic group optionally substituted by 1 to 5 halo atoms, n and t are each 0 or 1, r is 0, 1 or 2, the sum of n, r and t being between 0 and 3; $C_{1-4}$ alkoxy, $C_{1-4}$ carbalkoxy, one to 7 halo atoms, cyano, nitro or $C_{1-4}$ carbalkoxy provided that $R^{4a}$, $R^{4b}$, $R^{5a}$ and $R^{5b}$ are not all hydrogen and that one of $R^{4a}$, $R^{4b}$, $R^{5a}$ and $R^{5b}$ is not methyl when the others are hydrogen.

2) A compound according to claim 1 wherein $R^{2b}$ is a phenyl group substituted at the 3-,4- or 5-positions by one to three substituents each selected from halo, $C_{1-4}$ haloalkyl, $C_{1-4}$ haloalkoxy, $C_{1-4}$ haloalkylthio, cyano, or a group $(C{\equiv}C)_pR^9$ wherein p is 1 or 2 and $R^9$ is hydrogen, bromo, chloro, iodo or a group $S(O)_qR^{10}$ wherein q is 0, 1 or 2 and $R^{10}$ is trifluoromethyl, methyl or ethyl; or $R^9$ is an aliphatic group containing up to five carbon atoms optionally substituted by $C_{1-4}$ alkoxy, $C_{1-6}$ alkoxyalkoxy, $C_{1-8}$ acyloxy, halo or hydroxy or $R^9$ is a group $COR^{11}$ wherein $R^{11}$ is hydrogen, $C_{1-4}$ alkoxy, $C_{1-4}$ alkyl or a group $NR^{12}R^{13}$ wherein $R^{12}$ and $R^{13}$ are independently selected from hydrogen, methyl or ethyl; or $R^9$ is $SiR^{14}$, $R^{15}$, $R^{16}$ wherein $R^{14}$ and $R^{15}$ are the same or different and are each $C_{1-4}$ aliphatic groups and $R^{16}$ is a $C_{1-4}$ aliphatic group or phenyl provided that $R^{14}$, $R^{15}$ and $R^{16}$ do not contain more than 10 carbon atoms in total. The phenyl group is additionally optionally substituted at the 2- and/or 6-positions by fluoro or chloro.

3) A compound according to claim 1 wherein $R^{2b}$ is a group -A(C≡C)Z, wherein A is a $C_{3-5}$ aliphatic chain optionally containing a double bond and/or an oxygen atom and/or a group S(O)q wherein q is 0, 1 or 2 optionally substituted by halo, $C_{1-4}$ alkyl, $C_{1-4}$ haloalkyl, $C_{1-4}$ carbalkoxy or cyano and Z is hydrogen, $C_{1-5}$ alkyl, $C_{1-3}$ alkoxymethyl or a group $SiR^{14}$,$R^{15}$,$R^{16}$ wherein $R^{14}$, $R^{15}$ and $R^{16}$ are as hereinbefore defined.

4) A compound according to claim 1 wherein $R^{2b}$ is a group -B$Z^1$, wherein B is a group -$CH_2O$- or $CH_2S(O)q$ wherein q is 0, 1 or 2 or a $C_{2-3}$ aliphatic group each of which may be optionally substituted by one to three halo atoms and $Z^1$ is silyl substituted by three $C_{1-4}$ alkyl groups or $Z^1$ is a group

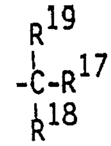

wherein $R^{17}$, $R^{18}$ and $R^{19}$ are the same or different and are each independently selected from halo, cyano, $C_{1-5}$ carbalkoxy, or a $C_{1-4}$ aliphatic group optionally substituted by halo,cyano, $C_{1-5}$ carbalkoxy, $C_{1-4}$ alkoxy or a group $S(O)_q R^{20}$ wherein $_q$ is 0, 1 or 2 and $R^{20}$ is $C_{1-4}$ alkyl, or $R^{17}$, $R^{18}$ and $R^{19}$ are selected from $C_{1-4}$ alkoxy or a group $S(O)_wR^{21}$ wherein w is 0, 1 or 2 and $R^{21}$ is $C_{1-4}$ alkyl optionally substituted by fluoro or $R^{17}$ and $R^{18}$ are linked to form a $C_{3-6}$ cycloalkyl ring, or one of $R^{17}$, $R^{18}$ and $R^{19}$ may be hydrogen.

5) A compound of the formula (I) according to claim 1 wherein $R^{2b}$ is a group

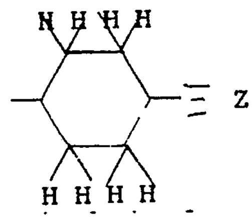

wherein Z is as hereinbefore defined:

6) A compound of the formula (I) according to any one of claims 1 to 5 wherein $R^{2a}$, $R^{4b}$ and $R^{5b}$ are each independently selected from hydrogen and methyl.

7) A compound of the formula (I) according to any one of claims 1 to 6 wherein $R^{4a}$ is selected from hydrogen and methyl, ethyl, isopropyl, isopropenyl, cyclopropyl, t-butyl and cyclobutyl groups and $R^{5a}$ is selected from methyl, ethyl, isopropyl, isopropenyl, cyclopropyl, t-butyl or cyclobutyl groups or $R^{4a}$ and $R^{5a}$ together with the carbon atoms of the dithiolane ring to which they are attached form a bridged ring system of the formula

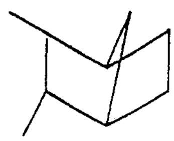

8) A process for the preparation of the compounds of the formula (I) according to any one of claims 1 to 7 which process comprises (i) the reaction of a compound of the formula (II):

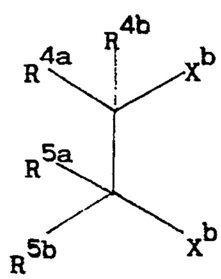

(II)

wherein $X^b$ is SH with a suitable aldehyde or ketone of the formula

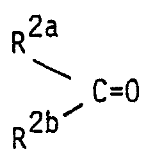

or a reactive derivative thereof, wherein $R^{2a}$, $R^{2b}$, $R^{4a}$, $R^{5a}$, $R^{4b}$ and $R^{5b}$ are as hereinbefore defined and, if required, thereafter oxidizing one or both of the ring sulphur atoms;

(ii) When $R^{2a}$ is hydrogen, the reaction of a dithiaborinane-dimethylsulphide complex of a compound of the formula (II) with a carboxylic acid

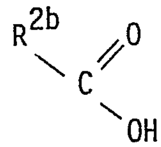

This reaction is carried out in the presence of a reducing agent such as stannous chloride in an inert solvent such as an ether, conveniently tetrahydrofuran, at a non-extreme temperature, for example between -20° and 100° and conveniently between 10° and 30°; and thereafter, if desired, converting one compound of the formula (I) to another compound of the formula (I) by methods well known to those skilled in the art.

9) An insecticidal or acaricidal composition comprising a compound of formula (I) as defined in any one of claims 1-7 in admixture with a carrier or diluent.

10) A synergised pesticidal composition comprising a compound of formula (I), as defined in any one of claims 1-7, a synergist for the formula (I) compound and a carrier or diluent.

11) A mixture of a compound of formula (I) as defined in any one of claims 1-7 and another pesticidal compound.

12) A method for the control of pests comprising application to the pest or to an environment susceptible to pest infestation of a pesticidally effective amount of a compound according to any one of claims 1-7 or a composition or mixture according to any one of claims 9-11.

13) A compound as defined in any one of claims 1-7 or composition according to any one of claims 9-11 for use in a method of surgery or therapy practised on the human or animal body or in a method of diagnosis practised on the human or animal body.

Claims for the following Contracting State: ES, GR

1) A process for the preparation of a compound of the formula (I)

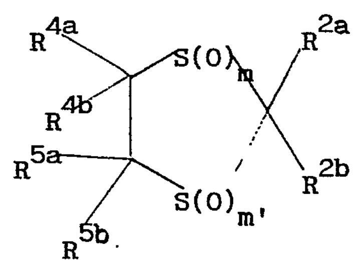

(I)

which contains between 10 and 27 carbon atoms, and wherein m and $m^1$ are independently selected from 0, 1 and 2; $R^{2a}$ is hydrogen, methyl, or ethyl; $R^{2b}$ is acetylene or contains between 3 and 18 carbon atoms and is a group $R^6$, wherein $R^6$ is a $C_{1-13}$ non-aromatic hydrocarbyl group, optionally substituted by a cyano or $C_{1-4}$ carbalkoxy group and/or by one or two hydroxy groups and/or by one to five halo atoms which are the same or different and/or by one to three groups $R^7$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^7$ and/or by a group -C≡CH, -C≡C-$R^6$ or C≡C-halo and/or by one to five halo atoms and/or by one to three $C_{1-4}$ haloalkyl groups wherein $R^6$ and $R^7$ are as hereinbefore defined; $R^{4b}$ and $R^{5b}$ are the same or different and are chosen from hydrogen, methyl or ethyl each optionally substituted by hydroxy, 1-5 fluoro atoms, methoxy, ethoxy, acetoxy, $C_{2-3}$ carbalkoxy or cyano or $R^{4b}$ and $R^{5b}$ and the carbon atoms of the dithiolane ring to which they are attached form a double bond; and $R^{4a}$ and $R^{5a}$ are the same or different and are each chosen from hydrogen or a $C_{1-11}$ non-aromatic hydrocarbyl group optionally substituted by 1 to 7 halo atoms, cyano, nitro, hydroxy, $C_{1-4}$ alkoxy or $C_{2-4}$ carbalkoxy groups or a phenyl or benzyl group optionally substituted by 1 to 5 halo atoms or $C_{1-4}$ haloalkyl groups, or $R^{4a}$ and $R^{5a}$ and the carbon atoms of the dithiolane ring to which they are attached form a cyclic or bridged polycyclic ring system containing between 3 and 9 atoms in the ring system and optionally substituted by a $C_{1-6}$ aliphatic group optionally substituted by 1 to 7 halo atoms or hydroxy or $C_{1-4}$ alkoxy groups; oxo, hydroxy a group $(O)_n$ $S(O)_r(O)_tR^8$ wherein $R^8$ is a $C_{1-4}$ aliphatic group optionally substituted by 1 to 5 halo atoms, n and t are each 0 or 1, r is 0, 1 or 2, the sum of n, r and t being between 0 and 3; $C_{1-4}$ alkoxy, $C_{1-4}$ carbalkoxy, one to 7 halo atoms, cyano, nitro or $C_{1-4}$

carbalkoxy provided that $R^{4a}$, $R^{4b}$, $R^{5a}$ and $R^{5b}$ are not all hydrogen and that one of $R^{4a}$, $R^{4b}$, $R^{5a}$ and $R^{5b}$ is not methyl when the others are hydrogen which process comprises (i) the reaction of a compound of the formula (II):

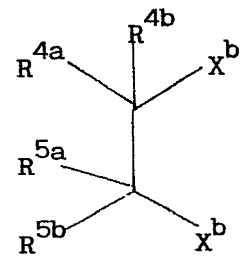

(II)

wherein $X^b$ is SH with a suitable aldehyde or ketone of the formula

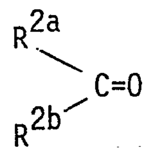

or a reactive derivative thereof, wherein $R^{2a}$, $R^{2b}$, $R^{4a}$, $R^{5a}$ , $R^{4b}$ and $R^{5b}$ are as hereinbefore defined and, if required, thereafter oxidizing one or both of the ring sulphur atoms;

(ii) When $R^{2a}$ is hydrogen, the reaction of a dithiaborinane-dimethylsulphide complex of a compound of the formula (II) with a carboxylic acid

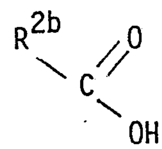

This reaction is carried out in the presence of a reducing agent such as stannous chloride in an inert solvent such as an ether, conveniently tetrahydrofuran, at a non-extreme temperature, for example between -20° and 100° and conveniently between 10° and 30°; and thereafter, if desired, converting one compound of the formula (I) to another compound of the formula (I) by methods well known to those skilled in the art.

2) A process according to claim 1 for the preparation of a compound wherein $R^{2b}$ is a phenyl group substituted at the 3-,4- or 5-positions by one to three substituents each selected from halo, $C_{1-4}$ haloalkyl, $C_{1-4}$ haloalkoxy, $C_{1-4}$ haloalkylthio, cyano, or a group $(C\equiv C)_pR^9$ wherein p is 1 or 2 and $R^9$ is hydrogen, bromo, chloro, iodo or a group $S(O)_qR^{10}$ wherein q is 0, 1 or 2 and $R^{10}$ is trifluoromethyl, methyl or ethyl; or $R^9$ is an aliphatic group containing up to five carbon atoms optionally substituted by $C_{1-4}$ alkoxy, $C_{1-6}$ alkoxyalkoxy, $C_{1-8}$ acyloxy, halo or hydroxy or $R^9$ is a group $COR^{11}$ wherein $R^{11}$ is hydrogen, $C_{1-4}$ alkoxy, $C_{1-4}$ alkyl or a group $NR^{12}R^{13}$ wherein $R^{12}$ and $R^{13}$ are independently selected from hydrogen, methyl or ethyl; or $R^9$ is $SiR^{14}$, $R^{15}$, $R^{16}$ wherein $R^{14}$ and $R^{15}$ are the same or different and are each $C_{1-4}$ aliphatic groups and $R^{16}$ is a $C_{1-4}$ aliphatic group or phenyl provided that $R^{14}$, $R^{15}$ and $R^{16}$ do not contain more than 10 carbon atoms in total. The phenyl group is additionally optionally substituted at the 2- and/or 6-positions by fluoro or chloro.

3) A process according to claim 1 for the preparation of a compound wherein $R^{2b}$ is a group $-A(C\equiv C)Z$, wherein A is a $C_{3-5}$ aliphatic chain optionally containing a double bond and/or an oxygen atom and/or a group S(O)q wherein q is 0, 1 or 2 optionally substituted by halo, $C_{1-4}$ alkyl, $C_{1-4}$ haloalkyl, $C_{1-4}$ carbalkoxy or cyano and Z is hydrogen, $C_{1-5}$ alkyl, $C_{1-3}$ alkoxymethyl or a group $SiR^{14}$,$R^{15}$,$R^{16}$ wherein $R^{14}$, $R^{15}$ and $R^{16}$ are as hereinbefore defined.

4) A process according to claim 1 for the preparation of a compound wherein $R^{2b}$ is a group $-BZ^1$, wherein B is a group $-CH_2O-$ or $CH_2S(O)q$ wherein $_q$ is 0, 1 or 2 or a $C_{2-3}$ aliphatic group each of which may be optionally substituted by one to three halo atoms and $Z^1$ is silyl substituted by three $C_{1-4}$ alkyl

groups or $Z^1$ is a group

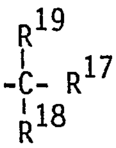

wherein $R^{17}$, $R^{18}$ and $R^{19}$ are the same or different and are each independently selected from halo, cyano, $C_{1-5}$ carbalkoxy, or a $C_{1-4}$ aliphatic group optionally substituted by halo,cyano, $C_{1-5}$ carbalkoxy, $C_{1-4}$ alkoxy or a group $S(O)_q$ $R^{20}$ wherein q is 0, 1 or 2 and $R^{20}$ is $C_{1-4}$ alkyl, or $R^{17}$, $R^{18}$ and $R^{19}$ are selected from $C_{1-4}$ alkoxy or a group $S(O)_wR^{21}$ wherein w is 0, 1 or 2 and $R^{21}$ is $C_{1-4}$ alkyl optionally substituted by fluoro or $R^{17}$ and $R^{18}$ are linked to form a $C_{3-6}$ cycloalkyl ring, or one of $R^{17}$, $R^{18}$ and $R^{19}$ may be hydrogen.

5) A process according to claim 1 for the preparation of a compound wherein $R^{2b}$ is a group

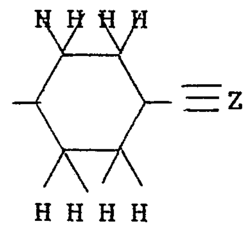

wherein Z is as hereinbefore defined:

6) A process according to any one of claims 1 to 5 for the preparation of a compound wherein $R^{2a}$, $R^{4b}$ and $R^{5b}$ are each independently selected from hydrogen and methyl.

7) A process according to any one of claims 1 to 6for the preparation of a compound wherein $R^{4a}$ is selected from hydrogen and methyl, ethyl, isopropyl, isopropenyl, cyclopropyl, t-butyl and cyclobutyl groups and $R^{5a}$ is selected from methyl, ethyl, isopropyl, isopropenyl, cyclopropyl, t-butyl or cyclobutyl groups or $R^{4a}$ and $R^{5a}$ together with the carbon atoms of the dithiolane ring to which they are attached form a bridged ring system of the formula

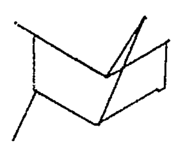

8) An insecticidal or acaricidal composition comprising a compound of formula (I) as defined in any one of claims 1-7 in admixture with a carrier or diluent.

9) A synergised pesticidal composition comprising a compound of formula (I), as defined in any one of claims 1-7, a synergist for the formula (I) compound and a carrier or diluent.

10) A mixture of a compound of formula (I) as defined in any one of claims 1-7 and another pesticidal compound.

11) A method for the control of pests comprising application to the pest or to an environment susceptible to pest infestation of a pesticidally effective amount of a compound according to any one of claims 1-7 or a composition or mixture according to any one of claims 8-10.

12) A compound as defined in any one of claims 1-7 or composition according to any one of claims 8-10 for use in a method of surgery or therapy practised on the human or animal body or in a method of diagnosis practised on the human or animal body.